# EUROPÄISCHE PATENTSCHRIFT

(11) **EP 3 324 769 B1**
(45) Veröffentlichungstag und Bekanntmachung des Hinweises auf die Patenterteilung: **16.02.2022**
(21) Anmeldenummer: 16747744.7
(22) Anmeldetag: 12.07.2016
(51) Int. Cl.: A41D 13/02, A41D 13/002, A41D 13/005, A41D 27/28, A62B 17/00, A41D 13/012

(54) **KÜHLANZUG**
COOLING SUIT
VÊTEMENT RÉFRIGÉRANT

(30) Priorität: 23.07.2015 CH 10752015
(43) Veröffentlichungstag der Anmeldung: 30.05.2018
(73) Patentinhaber: Beyeler, Patrick G., 1273 Arzier le Muids (CH)
(72) Erfinder: Beyeler, Patrick G., 1273 Arzier le Muids (CH)
(74) Vertreter: Felber, Josef
(86) Internationale Anmeldenummer: PCT/EP2016/066485
(87) Internationale Veröffentlichungsnummer: WO 2017/012908

(56) Entgegenhaltungen:
- DE-C1- 3 933 579
- DE-U- 1 822 905
- US-A- 2 573 414
- US-A- 4 738 119
- US-A- 5 492 108
- US-A1- 2006 174 392
- US-B1- 7 527 612

## Beschreibung

Die vorliegende Erfindung betrifft einen Anzug zum Kühlen des Körpers zum Arbeiten und für die Freizeit bei hohen Aussentemperaturen und/oder bei hoher Luftfeuchtigkeit, das heisst bei schwülem, drückenden Wetter.

Viele Aktivitäten in der Berufswelt oder auch bei Armeen und Schutzkräften erfolgen unter erschwerten Bedingungen, namentlich bei erhöhten Aussentemperaturen und/oder hoher Luftfeuchtigkeit. Das Verrichten von Arbeiten bei hohen Aussentemperaturen, insbesondere wenn die Luftfeuchtigkeit zusätzlich noch hoch ist, reduziert die Arbeitsleistung frappant. Bauarbeiter auf Baustellen in heissen Klimazonen sind nach kurzer Zeit erschöpft. Feuerwehrleute, die bei Bränden im Einsatz stehen, oder Rettungskräfte, in bei Katastrophen in heissen Zonen eingreifen müssen, leiden unter der grossen Hitze und kommen bald an die Grenze ihrer Leistungsfähigkeit. Ein weiteres Beispiel sind Piloten und Besatzungen von strahlgetriebenen Kampfflugzeugen während der Vorbereitungs- und Nachflugphase. Bis sie in den Genuss der bordeigenen Klimaanlage des Flugzeugs kommen, warten sie oft in einem Standby-Modus auf ihren Einsatz oder verrichten Vorbereitungsarbeiten ausserhalb des Flugzeuges. Um rasch einsatzfähig zu sein, tragen sie aber bereits die Einsatzbekleidung, das heisst eine volle Flieger- und/oder Kampfmontur. Diese ist schwer und erlaubt kaum eine Körperatmung. Sehr rasch kommt man darin ins Schwitzen und lange Phasen im Freien bei hohen Aussentemperaturen und/oder hoher Luftfeuchtigkeit werden zur Tortur. Aber auch Piloten und Besatzungsmitglieder von Helikoptern- sowie anderen Transport- und Sonderflugzeugen sind während der Vor- und Nachflugphase oftmals hohen Temperaturen und/oder einer hohen Luftfeuchtigkeit ausgesetzt und müssen dabei ihre spezielle Einsatzkleidung tragen, die geradezu dazu prädestiniert ist, einen Hitzestau herbeizuführen. Das gilt besonders auch während extremer Einsätze, bei denen die Flugzeuge und somit deren klimatisierte Kabinen - wenn diese überhaupt klimatisiert sind - verlassen werden müssen. Aber nicht nur Flugzeugbesatzungen, auch solche von Schiffen sowie Landfahrzeugen, welcher für spezielle Einsätze in heissen Klimazonen operieren, sind mit dem Problem der hohen Temperaturen und/oder der hohen Luftfeuchtigkeit konfrontiert. Im Grundsatz wird deren Leistungsfähigkeit bei hohen herrschenden Temperaturen und/oder bei hoher Luftfeuchtigkeit markant reduziert.

Gerade Einsatz-Spezialisten von Feuerwehren, Gefahrengut-Trupps oder Spezialeinheiten, die etwa zum Entschärfen von Blindgängern und Explosivkörpern entsandt werden, leiden besonders, wenn die Aussentemperaturen und/oder die Luftfeuchtigkeit hoch sind und sie unter Vollschutz mit entsprechender Spezial-Schutzkleidung arbeiten müssen. Aber auch Berufsgruppen, die unter extremen Bedingungen arbeiten, wie etwa Tunnelbauer, Bergwerkskumpel oder Giesser im Bereich von Hochöfen, oder Arbeiter an Ölbohrlöchern in heissen Wüstengebieten oder auf Bohrinseln in heissen Klimazonen sind hohen Temperaturen und oftmals schwüler Luft ausgesetzt und werden entsprechend beansprucht. Das Gleiche gilt auch für alle Land- und Waldarbeiter sowie Seeleute, die Arbeiten im Freien oder in Räumen und Bereichen erhöhter Temperatur und/oder Luftfeuchtigkeit verrichten müssen. Ihre Leistungsfähigkeit und ihr Wohlbefinden sind direkt abhängig von der herrschenden Temperatur und der aktuellen Luftfeuchtigkeit. Schliesslich betrifft diese Problematik auch Freizeitaktivitäten. Gartenarbeiten bei brütender Hitze und/oder schwülem Wetter sind sehr schweisstreibend und ermüdend, aber auch sportliche Aktivitäten im Freien, angefangen von Spaziergängen, Wanderungen, Lauftrainings bis hin zu Ballspielen und Radfahren werden zur Qual oder werden dann oftmals unterlassen, wenn das Wetter zu heiss oder zu schwül ist.

Bisher gibt es noch keine überzeugenden Lösungen für die Kühlung der Körper von Arbeits- und Einsatzpersonal, wenn dieses in heisser Umgebungstemperatur und/oder hoher Luftfeuchtigkeit aktiv sein muss. Die bekannten Vorschläge beinhalten meist elektrisch betriebene Kühlsysteme, zum Beispiel Schutzanzüge mit eingebauten elektrischen Mini-Ventilatoren, oder Anzüge mit Flüssigkeitskühlung, wobei die kühle Flüssigkeit durch Kanäle im Schutzanzug zirkuliert und extern über einen Kreislauf durch ein Kühlaggregat fliesst und darin gekühlt wird. Dieses ist für seinen Betrieb von elektrischem Strom abhängig oder mindestens von einem Verbrennungsmotor. Diese Lösung bedingt daher eine permanente Schlauchverbindung mit einem solchen relativ schweren externen Aggregat.

Das Dokument US 2003/0145946 A1 offenbart einen Kühlanzug, bestehend aus einem Anzug für Beine, Torso und Arme, der ab einer nach aussen mündenden Schlauchhülse nach innen verzweigende Gaskanäle mit mehreren Auslassöffnungen einschliesst, sowie einem Ausgangsschlauch zum Anschliessen an ein Kühlungsmittel, wobei der Anzug ein einteiliger Kombianzug ist, gefertigt aus einem textilen Material, auf dessen Innenseite die Gaskanäle angebracht sind, wobei der Kombianzug doppelwandig ausgeführt ist, mit einer Aussenschicht sowie einem gasdurchlässigen Innenfutter, und die Gaskanäle zwischen diesen beiden Schichten angeordnet sind, wobei die Gaskanäle in allen folgenden Bereichen mit Öffnungen zum Ausströmen Lassen von Gas versehen sind: für die Hüfte, den Bauch und die Brust.

Die bekannten Lösungen sind allesamt wenig praktikabel, umständlich in der Handhabung und aufwändig. Sie schliessen viele Komponenten ein, die zum Teil schwer sind und sie benötigen für ihren Einsatz eine externe Energiequelle. Ausserdem schlagen sich die vielen Komponenten in den hohen Anschaffungskosten eines solchen Kühlanzugs nieder. Der Betrieb und die Handhabung dieser Kühlanzüge sind darüber hinaus nicht als besonders einfach zu bezeichnen.

Angesichts dieser Situation ist es die Aufgabe der vorliegenden Erfindung, einen Kühlanzug für jedermann zu schaffen, der in heisser Umgebungsluft und/oder hoher Luftfeuchtigkeit arbeiten oder aktiv sein will oder muss, wobei dieser Kühlanzug leicht, effizient, einfach bedienbar, kostengünstig in der Anschaffung und im Unterhalt und narrensicher in seiner Funktion sein soll.

Diese Aufgabe wird gelöst von einem Kühlanzug nach Anspruch 1.

Im Gegensatz zu allen derzeit verfügbaren luftbetriebenen Körperkühlsystemen, die mit aus der Umgebung angesaugter warmer und/oder feuchter Luft arbeiten, wird dieser Kühlanzug mit einem trockenen, komprimierten Gas betrieben, das in einer Druckgasflasche mitgeführt wird. Strategisch platzierte flache Gaskanäle leiten das trockene und nach Entspannung kalte Gas an die verschiedenen ausgeprägten Schwitzstellen des Körpers, wodurch ein effizienter und physiologisch verträglicher Kühleffekt erzielt wird. Die Kühlung durch natürliche Konvektion wird durch das System unterstützt und der Schweiss an der Oberfläche durch die permanente Gaszufuhr über den atmungsaktiven Stoff abtransportiert.

Dieser Kühlanzug wird anhand der Zeichnungen als Ausführungsbeispiel offenbart und nachfolgend beschrieben. Seine Funktion und Handhabung wird erklärt und erläutert.

### Es zeigt:

- Figur 1:: Einen Träger des Kühlanzugs von vorne gesehen, mit den auf der Innenseite der Aussenhaut des Kühlanzugs angeordneten flachen Gaskanälen, die gestrichelt eingezeichnet sind, weil sie von aussen nicht sichtbar sind;
- Figur 2:: Den Träger des Kühlanzugs nach Figur 1 von hinten gesehen, mit den auf der Innenseite der Aussenhaut des Kühlanzugs angeordneten flachen Gaskanälen, die gestrichelt eingezeichnet sind, weil sie von aussen nicht sichtbar sind;
- Figur 3:: Den Träger des Kühlanzugs von schräg hinten gesehen, mit einer Druckgasflasche in einer Tragvorrichtung am Rücken tragend, und der Schlauchverbindung zu den Gaskanälen im Anzug.

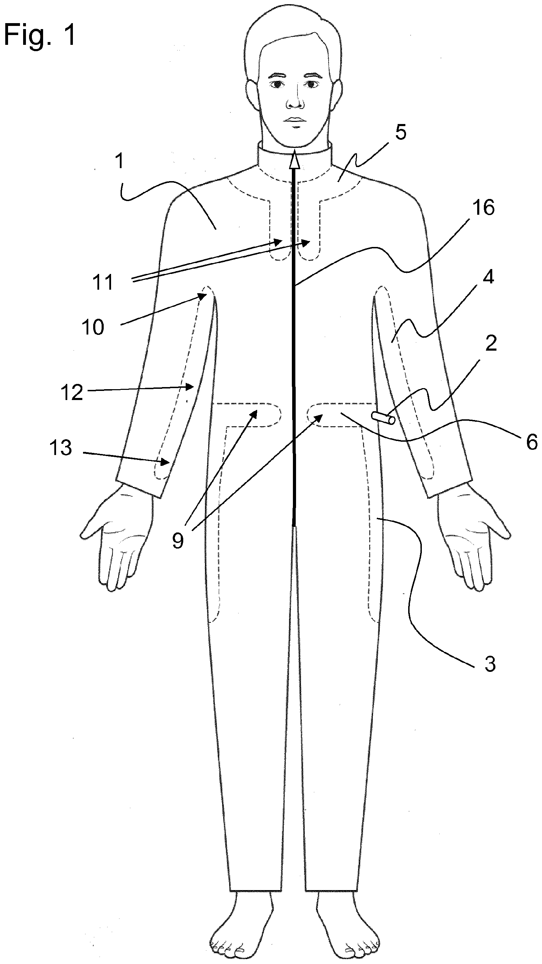

Der Kühlanzug wird in Figur 1 an einem Träger gezeigt, wobei dieser von vorne gesehen dargestellt ist. Der Kühlanzug ist ein Textilanzug, hier in Form eines sogenannten Kombi-Anzuges 1, das heisst einteilig mit Hosenbeinen und einem Oberteil für Torso und Arme ausgeführt. Zum bequemen Ein- und Ausstieg für das An- und Ausziehen ist der Anzug 1 mit einem Reissverschluss 16 ausgerüstet, welcher sich vom Schritt bis ganz nach oben zum Kragen hin erstreckt. Als Besonderheit ist dieser Anzug 1 auf der Innenseite seiner Aussenschicht mit verschiedenen Gaskanälen 3-6 versehen. Deren Verlauf ist hier gestrichelt eingezeichnet, weil man diese Gaskanäle 3-6 von aussen nicht sieht. Diese Gaskanäle 3-6 sind als flache Textilkanäle ausgeführt, die innen oder aussen mit einer gasundurchlässigen Schicht versehen sind, und die auf die Innenseite des Anzuges 1 angebracht sind, durch Aufkaschieren, Aufschweissen, oder sie sind an die Innenseite des Anzuges 1 angenäht oder auf die Innenseite aufgenäht.

Im gezeigten Beispiel beginnen diese Gaskanäle 3-6 als verzweigendes Kanalsystem bei einer Schlauchhülse 2 mit Kupplung, die im Hüftbereich seitlich aus dem Anzug 1 herausragt. Es ist indessen klar, dass diese Schlauchhülse auch anderswo aus dem Anzug ragen könnte, zum Beispiel im Brustbereich. Von der Schlauchhülse aus führt ein Gaskanal 6 in einen Ring, welcher die Hüfte umfasst, wobei der Ring im vorderen Bereich zum Öffnen des Reissverschlusses 16 offen bleibt. Ab diesem Ring zweigt je ein Gaskanal 3 vom Hüftbereich nach unten und ist auf der Aussenseite des Hosenbeines für den Oberschenkel ein stückweit nach unten geführt und führt weiter unten auf die Hinterseite des Hosenbeines, um schliesslich im Kniehöhlenbereich zu enden.

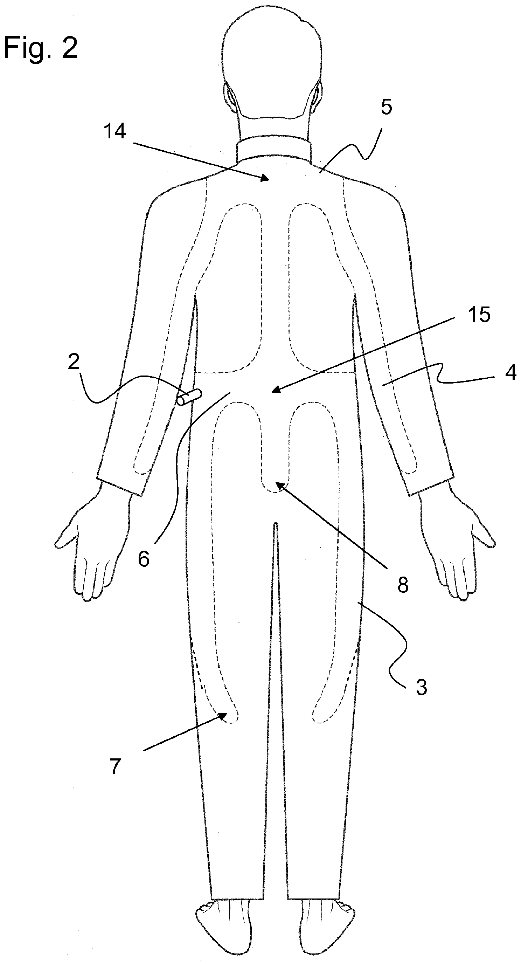

Anhand von Figur 2, welche diesen Träger des Kühlanzugs nach Figur 1 von hinten gesehen zeigt, erschliesst sich der weitere Verlauf der Gaskanäle. Vom Ring, der die Hüfte umschliesst, zweigt ein Gaskanal aus dem Bereich des Kreuzes 15 nach oben ab und verläuft längs der Wirbelsäule in einen weiteren, ringförmig angelegten Gaskanal 5, welcher den Nacken und Hals des Trägers umschliesst. Auch dieser Ring ist vorne offen, wie man in Figur 1 erkennt, damit der durchgehende Reissverschluss 16 geöffnet werden kann. Ab dem Ring verlaufen auf der Vorderseite des Anzuges zwei Abschnitte längs des Reissverschlusses 16 nach unten und enden im Brustbereich 11 des Trägers. Ab dem ringförmigen Gaskanal 5 zweigt weiter je ein Gaskanal 4 über die Schultern in die Ärmel des Anzugs ab. Diese beiden Gaskanäle 4 verlaufen längs der Ärmel, vorzugsweise auf jener Seite der Ärmel, die der Innenseite der Arme des Trägers gegenüber liegen. Schliesslich gibt es einen vom Ring an der Hüfte von hinten in der Mitte nach unten abzweigenden Gaskanal, der nach unten in den Gesässbereich führt und im Bereich des Schrittes 8 endet.

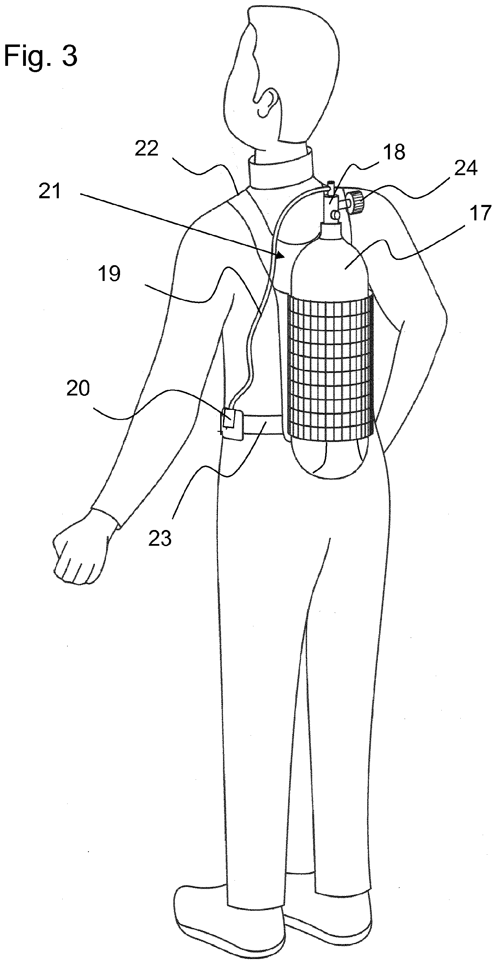

Diese verschiedenen Gaskanäle 3-6 werden alle über die Schlauchhülse 2 und durch einen angekuppelten Schlauch aus einer Druckgasflasche mit daraus bezogenem entspannten Gas versorgt, wie das später anhand von Figur 3 klarwird. Das durch die Entspannung abgekühlte Gas, im einfachsten Fall gewöhnliche Luft, strömt ab der Schlauchhülse 2 in das gesamte verzweigende Gaskanäle-System und wird an ganz bestimmten Stellen jeweils zu einem Teil zum Ausströmen gebracht, wobei das restliche Gas im Kanalsystem weiterströmt. Diese Ausström-Stellen sind in den Figuren 1 und 2 mit Pfeilen eingetragen. Es sind jene Stellen, an denen der Körper vor allem transpiriert und wo er effizient Wärme abführen kann. Eine erste Auslassöffnung befindet sich im Bereich des Kreuzes 15 des Trägers und auf der Vorderseite des die Hüfte umschliessenden Ringes im Bereich des Bauches 9. Eine nächste Auslassöffnung befindet sich im Bereich des Nackens 14 und im Bereich der Brust 11. Die Gas- oder Luftkanäle 4, welche über die Schultern in die Ärmel geführt sind, weisen im Bereich der Achselhöhlen 10 Auslassöffnungen auf, sowie im Bereich der Armgelenke 12, das heisst auf der Innenseite der Gelenke, und schliesslich im vorderen Bereich der Ärmel auf jener Seite, die der inneren Seite 13 des Unterarms des Trägers gegenüberliegt. Gerade aus dem Bereich des Innenarmes 13 des Unterarmes kann sehr viel Wärme abgeführt werden, weil dort das Blut in Adern, Venen und Gefässen zirkuliert, die nahe an der Hautoberflächen verlaufen. Im unteren Bereich des Körpers sind Gasauslässe im Bereich der Kniehöhlen 7 angeordnet, und ausserdem am Ende des Gaskanals, welcher entlang des Gesässes zum Bereich des Schrittes 8 führt, wo ebenfalls eine Auslassöffnung angeordnet ist. Der grösste Druck herrscht im Gaskanalsystem herrscht im Abschnitt, bevor das Gas eine erste Auslassöffnung erreicht, das heisst im Ring, welcher die Hüfte umschliesst. Nach jeder Passage einer Auslassöffnung wird der Gasdruck reduziert und ist schliesslich in den Bereichen der Innenarme der Vorderarme sowie in den Kniehöhlen am geringsten. Damit das Kühlgas oder die Kühlluft wunschgemäss verteilt wird, müssen die Auslassöffnungen gegen die äusseren Enden des verzweigenden Gaskanalsystems hin immer etwas grösser werden, um dem Druckabfall Rechnung zu tragen. Im Bereich des Kreuzes ist die Auslassöffnung am kleinsten dimensioniert, weil dort der Druck im Gaskanalsystem noch am grössten ist, und im Bereich der Vorderarme sind die Auslassöffnungen grösser bemessen. Dadurch lässt sich der Druckabfall kompensieren, sodass je nach Grösse und Gestaltung der Auslassöffnungen überall etwa gleichviel Gas bzw. Luft pro Zeit ausströmt. Die Auslassöffnungen können als Schlitze oder als feine Perforationen in den Gaskanälen 4-6 gebildet sein.

In einer Variante kann das Gaskanal-System in eine Anzahl separierter Gaskanäle für gewisse oder für jede der Auslassöffnungen unterteilt sein, sodass also der Gasdruck in jedem Kanal gleich verläuft und jede Auslassöffnung mit demselben Druck bedienbar ist. Dann aber muss jeder getrennte Gaskanal separat mit Druckgas aus der Druckgasflasche versorgt werden. Es kann dann auch jeder Schlauch mit einem gesonderten Ventil ausgerüstet sein, sodass jede Auslassöffnung mit einstellbarem Druck bedient werden kann.

In einer weiteren Variante kann der Anzug auch als ein zweiteiliger Anzug aus Hose und Jacke aus einem textilen Material hergestellt sein. Die verzweigenden Gaskanäle sind dann über eine zusätzliche Schlauchkupplung zwischen Hose und Jacke verbindbar. Beide Kleidungstücke, das heisst Jacke und Hose können auch unabhängig voneinander mit einem solchen Kühlsystem ausgerüstet sein, also eines für die Hose mit einem eigenen Zufuhrschlauch und eines für die Jacke mit ebenfalls einem eigenen Zufuhrschlauch.

Die Gaskanäle sind wie bisher beschrieben auf der Innenseite der Aussenschicht des Anzugs 1 angebraucht. Der Anzug ist mit einem Innenfutter ausgestattet, welches diese Gaskanäle 3-6 überdeckt, sodass überall nur das Innenfutter am Körper des Trägers anliegt. Dieses ist aus einem gut hautverträglichen, atmungsaktiven und auch Schweiss absorbierenden textilen Material herstellt, zum Beispiel aus reiner Baumwolle oder aus Baumwolle mit nur einem geringen Anteil synthetischer Fasern. Ein Baumwoll-Innenfutter macht den Anzug komfortabel zum Tragen und ausserdem ist ein solches Innenfutter von dem an den Austrittsöffnungen ausströmenden Gas durchströmbar, und dieses sucht sich dann ganz frei längs des Körpers und Anzuges einen Weg nach aussen und strömt schliesslich vorwiegend an den Ärmelöffnungen, am Nacken sowie an den unteren Enden der Hosenbeine nach aussen.

Die Figur 3 zeigt den Träger des Kühlanzugs von schräg hinten gesehen. Er trägt eine Druckgasflasche 17 in einer Tragvorrichtung 21 am Rücken mit. Diese Flasche 17 enthält zum Beispiel 3kg Druckluft oder ein anderes geeignetes, trockenes Gas, zum Beispiel Stickstoff, auf einem Druck von zum Beispiel 300 bar. Damit bildet diese Flasche 17 und ihr Druckluftinhalt einen Energiespeicher, und genau diese Energie wird zum effizienten Kühlen des Körpers des Trägers eingesetzt. Durch das dosierte Ausströmen Lassen des Druckgases bzw. der Druckluft wird dieses beim Ausströmen adiabatisch entspannt. Die in Form von Druck gespeicherte Energie wird in Form von Wärme an die Umgebung abgeführt, wodurch das Gas bzw. die eine erhebliche Abkühlung erfährt. Die auf diese Weise völlig passiv abgekühlte Luft oder entsprechend das Kühlgas strömt dann über die Schlauchkupplung 20 in das Gaskanalsystem des Anzugs und tritt nach und nach durch verschiedenen Auslassöffnungen in diesem Gaskanalsystem aus. Die Luft oder das Gas nimmt Wärme vom Körper des Trägers auf bzw. kühlt in an den besagten Stellen in physiologisch verträglichem und fein einstellbaren Mass. Die Kühlung durch natürliche Konvektion wird durch das System unterstützt und der Schweiss an der Oberfläche des Körpers des Trägers wird von der permanente vorbeiströmende Luft bzw. dem Gas aufgenommen und über den atmungsaktiven Stoff des Innenfutters schliesslich nach aussen abtransportiert.

Die Druckgasflasche 17 ist mit einem Ventil 18 ausgerüstet, das in mehreren einrastbaren Stellungen mittels eines Stellrades 24 öffnenbar ist. Je nach dem Grad der Öffnung des Ventils 18 strömt mehr oder weniger Druckgas pro Zeit aus der Druckgasflasche 17. Je mehr Druckgas entlassen wird, umso grösser ist die Kühlwirkung im Anzug, aber je kürzer wird die Einsatzdauer. Mit einer Druckgasflasche von 2 Litern Volumen und zum Beispiel Luft auf einem Druck von 300bar kann 600 Liter Luft mitgetragen werden. Eine Druckflasche mit 3 Litern Volumen enthält bei 600bar sogar 900 Liter komprimierte Luft. In der geringsten Öffnungsstellung strömt ca. 1 Liter Luft pro Minute stetig aus der Flasche, sodass man eine maximale Ausströmdauer von 10 Stunden erreicht. Bei höherer Kühlleistung kann bis zu 20 Liter Luft pro Minute aus der Flasche bezogen werden, wobei dann die Kühldauer auf 30 Minuten reduziert ist. Eine Schnellkühlfunktion erlaubt die Freigabe von 40 Litern Luft bzw. Gar pro Minute. In diesem Modus lässt sich ein Körper während 15 Minuten intensiv kühlen, wenn etwa ein Einsatz nahe eines Brandherdes nötig ist.

Der Träger kann jederzeit durch Einstellen des Ventils 18 die Kühlleistung auf ein Mass einstellen, das ihm angenehm ist. Es ist klar, dass dieses Ventil 18 mit Stellrad 24 auch anderswo als direkt an der Flasche 17 angeordnet sein kann, zum Beispiel an einem mit dem Anzug getragenen Gürtel, oder vorne an der Trägervorrichtung 12, damit das Stellrad 24 in Griffweite ist und auch sichtbar. In diesem Fall führt ein Schlauch von der Flasche 17 zum Ventil 24, und vom Ventil hernach zur Schlauchhülse 2. Ausserdem kann die Druckflasche 17 optional mit einem Manometer ausgerüstet sein, womit Rückschlüsse auf den aktuellen Flascheninhalt gezogen werden können, sodass man jederzeit weiss, wie lange man noch über eine intakte Kühlfunktion verfügt. Es versteht sich, dass die minimale und maximale Kühldauer vom Druckgasinhalt und dem darin herrschenden Druck abhängig ist. Eine Druckflasche mit 2 Litern Fassungsvermögen oder noch mehr Gas- oder Luftinhalt bietet noch längere Kühlzeiten.

Um möglichst wenig Gewicht herumtragen zu müssen, ist die Druckgasflasche 17 vorzugsweise aus Leichtbaumaterial gefertigt, zum Beispiel auf Basis von kohlenstoffverstärkten Fasern und mit einer luftdichten Beschichtung versehen. Solche Flaschen sind handelsüblich. Ausserdem ist die Druckgasflasche 17 in eine massgeschneiderte Trägervorrichtung 21 einsetzbar, mit welcher sie sehr komfortabel am Rücken mitgetragen werden kann. Hierfür ist die Trägervorrichtung mit zwei breiten Schulter-Trägerschlaufen 22 ausgerüstet, sowie mit einem Beckengurt 23. Mit dieser Trägervorrichtung 21 kann sich der Träger auch mühelos bücken, oder er kann niederknien und auch auf die Seite liegen, um Arbeiten zu verrichten. Die Vorrichtung ist kompakt und leicht und stört ihn beim Verrichten seiner Arbeiten nicht oder kaum. Aufgrund ihres geringen Gewichtes muss der Träger mit dem gesamten System bloss ein Mehrgewicht von ungefähr 5kg mit sich tragen, dafür kann er sich in perfekten körperklimatischen Bedingungen betätigen. Der Anzug alleine wiegt ca. 1.5kg. Die Trägervorrichtung 21 mitsamt einer zum Beispiel 2 Liter Druckflasche bringt ein Gewicht von bloss insgesamt weniger als 6.5kg auf die Waage.

Wird die Druckgasflasche bzw. Druckluftflasche während eines Einsatzes leer, so kann sie mit wenigen Handgriffen durch eine gefüllte ersetzt werden. Hierzu wird die Schlauchverbindung an der Schlauchhülse 2 gelöst, die Druckflasche 17 aus der Trägervorrichtung 21 entnommen und eine gefüllte Druckflasche 17 wird eingesetzt. Deren Schlauch 19 wird mit der Schlauchhülse 2 am Kühlanzug 1 gekuppelt und schon ist das Kühlsystem wieder einsatzbereit. Als Schlauchkupplung eignet sich eine handelsübliche Schnellkupplung für Gasschläuche, zum Beispiel eine Schwenkkupplung für druckloses Kuppeln oder eine Linear-Kupplung.

Dieser Kühlanzug kann aber nicht nur mit einer direkt mitgetragenen Druckflasche benützt werden, sondern mit Druckluft aus einer gesondert aufgestellten Druckflasche oder einem gesondert vorhandenen Druckbehälter, besonders dann, wenn die Benützer sitzend auf Sitzen ihre Tätigkeit verrichten, etwa zum Fliegen oder Mitfliegen in Helikoptern oder Flugzeugen und zum Fahren und Mitfahren in Fahrzeugen aller Art. In dieser Weise eingesetzt eignet er sich zum Einsatz zum Beispiel in Helikoptern, Transportflugzeugen, Panzern, Zivil- und Militärfahrzeugen, Schiffen, U-Booten etc., wobei dann die entspannte Druckluft aus einer beigestellten Druckflasche oder einem stationär eingebauten Druckbehälter bezogen werden kann. Es können dann auch mehrere Kühlanzüge kollektiv an einen grossen Druckbehälter angeschlossen werden, welcher von einem bordeigenen Kompressor nachgeladen werden kann, wobei dann der Druckbehälter separat aktiv von einer Kühleinrichtung gekühlt werden kann, zum Beispiel auf die normale Innentemperatur des betreffenden Flugzeuges, Fahrzeuges oder sonstigen Raumes. Dieser Kühlanzug bietet daher für sehr viele Mitglieder von Einsatzkräften aller Art eine enorme Erleichterung ihrer Arbeitsbedingungen und er steigert deren Arbeitseffizienz ganz entscheidend. Er kann aber auch von jeder Privatperson eingesetzt werden, für jedwede Arbeiten oder sportliche Aktivitäten im Freien, wenn immer die herrschenden Temperaturen hoch sind oder eine hohe Luftfeuchtigkeit zu schwülen Wetterbedingungen führt.

### Ziffernverzeichnis

- 1: Anzug
- 2: Schlauchhülse
- 3: Luftkanal in den Hosenstoss
- 4: Luftkanal in den Ärmel
- 5: Luftkanal im Schulterbereich
- 6: Luftkanal um die Hüfte
- 7: Kniehöhlenbereich
- 8: Schritt
- 9: Bauchbereich
- 10: Achselhöhlenbereich
- 11: Brustbereich
- 12: Armgelenkbereich
- 13: Innenseite des Vorderarms
- 14: Nackenbereich
- 15: Kreuzbereich
- 16: Reissverschluss am Kombi
- 17: Druckluftflasche
- 18: Ventil an Druckluftflasche
- 19: Schlauch ab Druckluftflasche
- 20: Schlauchkupplung
- 21: Trägervorrichtung für Druckluftflasche
- 22: Trägerschlaufen zur Trägervorrichtung
- 23: Hüftgurt zur Trägervorrichtung
- 24: Stellrad für Druckluftventil

## Patentansprüche

1. Kühlanzug, bestehend aus einem Anzug (1) für Beine, Torso und Arme, der ab einer nach außen mündenden Schlauchhülse (2) nach innen verzweigende Gaskanäle (3-6) mit mehreren Auslassöffnungen einschließt, sowie einer zugehörigen Druckgasflasche (17) zum Mittragen oder einem zugehörigen stationär aufstellbaren Druckbehälter zum Anschließen eines Ausgangsschlauches (19) mit Kupplungsstück (20) an die Schlauchhülse (2), wobei ein Ventil (18) mit Stellrad (24) an der Druckgasflasche (17) oder am Anzug vorhanden ist, zur dosierten Abgabe von entspanntem Gas aus der Druckgasflasche (17) oder dem stationären Druckbehälter in diese Gaskanäle (3-6),
wobei der Anzug entweder ein einteiliger Kombianzug mit Reißverschluss (16) zum An- und Ausziehen ist, gefertigt aus einem textilen Material, auf dessen Innenseite die Gaskanäle (3-6) angebracht sind,
oder ein zweiteiliger Anzug aus Hose und Jacke aus einem textilen Material ist, wobei die verzweigenden Gaskanäle (3-6) über eine zusätzliche Schlauchkupplung zwischen Hose und Jacke verbindbar sind,
oder ein zweiteiliger Anzug aus Hose und Jacke aus einem textilen Material ist, wobei die verzweigenden Gaskanäle (3-6) für Hose und Jacke je separat über eine Schlauchkupplung (23) mit einer Druckluftflasche (17) verbindbar sind, so dass Jacke und Hose unabhängig voneinander mit je einem eigenen Kühlsystem kühlbar sind,
wobei der Kombianzug oder die Jacke und Hose doppelwandig ausgeführt sind, mit einer Außenschicht sowie einem gasdurchlässigen Innenfutter, und die Gaskanäle (3-6) zwischen diesen beiden Schichten angeordnet sind, wobei sie in einzelnen oder allen folgenden Bereichen mit Öffnungen zum Ausströmen Lassen von Gas versehen sind: für die Hüfte (6), die Kniehöhlen (7), den Schritt (8), den Bauch (9), die Achselhöhlen (10), die Brust (11), die Armgelenke (12), die Innenseite der Vorderarme (13), den Nacken (14) und das Kreuz (15) und
wobei die Gaskanäle (3-6) aus flachen Kanälen gebildet sind, welche auf der Innenseite der Außenschicht des Anzugs befestigt sind.

2. Kühlanzug nach Anspruch 1, wobei die Gaskanäle (3-6) vom Bereich des Kreuzes (15) des Anzugs (1) aus nach oben und unten verzweigen, nämlich nach unten in die beiden Hosenbeine und über das Gesäß zum Schritt, und nach oben zum Bereich des Nackens (14) hin sowie mit je einem Kanal zu den Achselhöhlen (10) und in die Ärmel.

3. Kühlanzug nach einem der vorangehenden Ansprüche, wobei die Gaskanäle im Oberteil des Anzuges Ausströmöffnungen im Bereich des Kreuzes (15), an den Hüften und im Bereich des Nackens (14), in den Achselhöhlen (10), an den Innenseiten des Armgelenkes (12) sowie den Innenseiten (13) der Ärmel aufweisen, und im Unterteil, das heißt in den Hosenbeinen Ausströmöffnungen in den Kniehöhlen (7) sowie im Bereich des Schrittes (8) angeordnet sind.

4. Kühlanzug nach einem der vorangehenden Ansprüche, wobei die Druckgasflasche (17) aus Karbonfasermaterial gefertigt ist und mit einem textilen Trägersystem (21) ausgerüstet ist, zum Tragen der Druckgasflasche (17) am Rücken nach der Art eines Rucksackes.

5. Kühlanzug nach Anspruch 4, wobei die Druckgasflasche (17) aus Karbonfasermaterial gefertigt ist und ein Volumen von 2 bis 5 Litern fasst, zum Mitführen von Druckluft auf einem Druck von 300bar von 600 Litern bis 1500 Litern.

6. Verwendung eines Kühlanzuges nach einem der vorhergehenden Ansprüche zum Zweck der individuellen Körperkühlung für fliegendes und fahrendes Personal, insbesondere für Personal in Helikoptern, Flugzeugen, Panzern, Fahrzeugen, Schiffen und U-Booten.

7. Verwendung eines Kühlanzuges nach einem der Ansprüche 1 bis 5 zum Zweck der individuellen Körperkühlung für im Freien bei heissen Temperaturen oder in Industriebetrieben mit Zonen hoher Raumtemperatur arbeitendes Personal.

## Claims

1. Cooling suit, consisting of a suit (1) for legs, torso and arms, which from a hose sleeve (2) opening outwards includes inwardly branching gas ducts (3-6) with a plurality of outlet openings, as well as an associated compressed gas bottle (17) for carrying along or an associated pressure container, which can be set up in a stationary manner, for connecting an outlet hose (19) with coupling piece (20) to the hose sleeve (2), wherein a valve (18) with an adjusting wheel (24) is provided on the compressed gas cylinder (17) or on the suit, for the metered discharge of expanded gas from the compressed gas cylinder (17) or the stationary pressure container into these gas ducts (3-6), wherein the suit is either a one-piece combination suit with a zip fastener (16) for putting on and taking off, made of a textile material, on the inside of which the gas ducts (3-6) are arranged or is a two-piece suit consisting of trousers and jacket made of a textile material, wherein the branching gas ducts (3-6) can be connected via an additional hose coupling between the trousers and jacket, or is a two-piece suit consisting of trousers and jacket made of a textile material, wherein the branching gas ducts (3-6) for trousers and jacket can each be connected separately via a hose coupling (23) to a compressed air cylinder (17), so that the jacket and trousers can be cooled independently of one another, each with their own cooling system, the combination suit or the jacket and trousers being of double-walled construction, with an outer layer and a gas-permeable inner lining, and the gas ducts (3-6) being arranged between these two layers, being provided in individual or in all of the following regions with openings for letting out gas: for the hip (6), the knee cavities (7), the crotch (8), the abdomen (9), the armpits (10), the chest (11), the arm joints (12), the inside of the front arms (13), the neck (14) and the cross (15) and wherein the gas channels (3-6) are formed of flat channels which are fixed to the inside of the outer layer of the suit.

2. Cooling suit according to claim 1, wherein the gas ducts (3-6) branch upwards and downwards from the region of the cross (15) of the suit (1), namely downwards into the two trouser legs and over the buttocks to the crotch, and upwards towards the region of the neck (14) as well as with one duct each to the armpits (10) and into the sleeves.

3. Cooling suit according to one of the preceding claims, wherein the gas channels in the upper part of the suit have outflow openings in the region of the cross (15), at the hips and in the region of the neck (14), in the armpits (10), on the inner sides of the arm joint (12) as well as the inner sides (13) of the sleeves, and in the lower part, i.e. in the trouser legs, outflow openings are arranged in the knee cavities (7) as well as in the region of the crotch (8).

4. Cooling suit according to one of the preceding claims, wherein the pressurised gas bottle (17) is made of carbon fibre material and is equipped with a textile carrier system (21) for carrying the pressurised gas bottle (17) on the back in the manner of a backpack.

5. Cooling suit according to claim 4, wherein the compressed gas bottle (17) is made of carbon fibre material and has a volume of 2 to 5 litres, for carrying compressed air at a pressure of 300 bar of 600 litres to 1500 litres.

6. Use of a cooling suit according to any of the preceding claims for the purpose of individual body cooling for flying and travelling personnel, in particular for personnel in helicopters, aircraft, tanks, vehicles, ships and submarines.

7. Use of a cooling suit according to any one of claims 1 to 5 for the purpose of individual body cooling for personnel working outdoors at hot temperatures or in industrial plants with zones of high room temperature.

## Revendications

1. Combinaison réfrigérante, constituée d'une combinaison (1) pour les jambes, le torse et les bras, qui comprend, à partir d'une douille tubulaire (2) débouchant vers l'extérieur, des canaux de gaz (3-6) se ramifiant vers l'intérieur et présentant plusieurs ouvertures de sortie, ainsi que d'une bouteille de gaz comprimé (17) correspondante destinée à être portée ou d'un récipient sous pression correspondant, pouvant être installé de manière stationnaire, pour le raccordement d'un tuyau de sortie (19) avec pièce d'accouplement (20) à la douille tubulaire (2), une soupape (18) avec roue de réglage (24) étant présente sur la bouteille de gaz comprimé (17) ou sur la combinaison, pour la distribution dosée de gaz détendu de la bouteille de gaz comprimé (17) ou du récipient sous pression stationnaire dans ces canaux de gaz (3-6), la combinaison étant soit une combinaison combinée d'une seule pièce avec fermeture à glissière (16) pour l'habillage et le déshabillage, fabriquée en un matériau textile sur la face intérieure duquel sont appliqués les canaux de gaz (3-6), ou une combinaison en deux parties composée d'un pantalon et d'une veste en matériau textile, les canaux de gaz (3-6) ramifiés pouvant être reliés entre le pantalon et la veste par un raccord de tuyau supplémentaire, ou une combinaison en deux parties composée d'un pantalon et d'une veste en matériau textile, les canaux de gaz (3-6) ramifiés pour le pantalon et la veste pouvant être reliés chacun séparément à une bouteille d'air comprimé (17) par un raccord de tuyau (23), de sorte que la veste et le pantalon peuvent être refroidis indépendamment l'un de l'autre, chacun avec son propre système de refroidissement, la combinaison ou la veste et le pantalon étant réalisés à double paroi, avec une couche extérieure ainsi qu'une doublure intérieure perméable aux gaz, et les canaux de gaz (3-6) étant disposés entre ces deux couches, en étant pourvus, dans certaines zones ou dans toutes les zones suivantes, d'ouvertures pour laisser s'échapper le gaz: pour les hanches (6), les creux des genoux (7), l'entrejambe (8), le ventre (9), les aisselles (10), la poitrine (11), les articulations des bras (12), l'intérieur des avant-bras (13), la nuque (14) et la croix (15) et où les canaux de gaz (3-6) sont formés par des canaux plats qui sont fixés sur la face intérieure de la couche extérieure du vêtement.

2. Combinaison réfrigérante selon la revendication 1, dans laquelle les canaux de gaz (3-6) se ramifient vers le haut et vers le bas à partir de la zone de la croix (15) de la combinaison (1), à savoir vers le bas dans les deux jambes de pantalon et par-dessus les fesses vers l'entrejambe, et vers le haut vers la zone de la nuque (14) ainsi que par un canal vers les aisselles (10) et dans les manches.

3. Combinaison réfrigérante selon l'une des revendications précédentes, dans laquelle les canaux de gaz dans la partie supérieure de la combinaison présentent des ouvertures de sortie dans la zone de la croix (15), sur les hanches et dans la zone de la nuque (14), dans les aisselles (10), sur les côtés intérieurs de l'articulation du bras (12) ainsi que sur les côtés intérieurs (13) des manches, et dans la partie inférieure, c'est-à-dire dans les jambes du pantalon, des ouvertures de sortie sont disposées dans les creux des genoux (7) ainsi que dans la zone de l'entrejambe (8).

4. Combinaison réfrigérante selon l'une des revendications précédentes, dans laquelle la bouteille de gaz comprimé (17) est fabriquée en matériau à base de fibres de carbone et est équipée d'un système de support textile (21), pour porter la bouteille de gaz comprimé (17) sur le dos à la manière d'un sac à dos.

5. Combinaison réfrigérante selon la revendication 4, dans laquelle la bouteille de gaz comprimé (17) est fabriquée en matériau à base de fibres de carbone et a une capacité de 2 à 5 litres, pour transporter de l'air comprimé à une pression de 300 bars de 600 litres à 1500 litres.

6. Utilisation d'une combinaison réfrigérante selon l'une des revendications précédentes dans le but de refroidir individuellement le corps du personnel volant et navigant, en particulier le personnel des hélicoptères, des avions, des chars d'assaut, des véhicules, des navires et des sous-marins.

7. Utilisation d'une combinaison réfrigérante selon l'une des revendications 1 à 5 dans le but de refroidir individuellement le corps du personnel travaillant à l'extérieur par des températures chaudes ou dans des entreprises industrielles comportant des zones de température ambiante élevée.
